# EUROPEAN PATENT APPLICATION

(11) **EP 4 840 336 A1**
(43) Date of publication of application: **18.11.2026**
(21) Application number: 25175639.1
(22) Date of filing: 12.05.2025
(51) Int. Cl.: A24F 40/46, H03K 3/84, H03K 7/08, H02M 3/156, H02M 3/158, A61M 15/06, A61M 11/04, H02M 1/44, A24F 40/50

(54) **AEROSOL-GENERATING SYSTEM**

(71) Applicant: Imperial Tobacco Limited, Bristol BS3 2LL (GB)
(72) Inventor: HYLA, Jan, Bristol, BS3 2LL (GB)
(74) Representative: Mewburn Ellis LLP

(57) **Abstract**

There is provided an aerosol-generating system that comprising an aerosol-generating unit configured to generate an aerosol from an aerosol-forming material; a power source configured to supply electrical energy to the aerosol-generating unit; and a controller configured to control the supply of electrical energy from the power source to the aerosol-generating unit to supply a random pulse-width modulated voltage signal to the aerosol-generating unit, the random pulse-width modulated voltage signal comprising a plurality of voltage pulses and being characterized by one or more randomly generated parameters. There is also provided a method of performing power control on an aerosol-generating system, the aerosol-generating system.

## Description

### FIELD

The present disclosure relates to an aerosol-generating system and a method of performing power control on an aerosol-generating system.

### BACKGROUND

An electronic vapour product (EVP), also known as a vaping apparatus or a vape, is a type of aerosol-generating system in which an aerosol precursor (e.g. a liquid or gel) is aerosolised by an aerosol-generating unit, such as a heating element or ultrasonic element.

A heat-not-burn (HNB) device, also known as a heated tobacco device, is a type of aerosol-generating system in which an aerosol precursor (e.g., a solid precursor such as tobacco) is heated by a heating system to produce an aerosol that can be inhaled by the user.

Aerosol-generating systems including EVPs and HNB devices typically have an aerosol-generating unit which generates an aerosol from an aerosol-forming material. The aerosol-generating unit is powered by a power source. Accurate and precise control of the power supplied from the power source to the aerosol-generating unit is important to ensure reliable operation of the system within predetermined operational limits.

It is against this background that the present invention has been developed.

### SUMMARY

At its most general, the present invention relates to supplying a random pulse-width modulated voltage signal to the aerosol-generating unit of an aerosol-generating system.

In a first aspect, the present disclosure provides an aerosol-generating system that comprises:
an aerosol-generating unit configured to generate an aerosol from an aerosol-forming material;
a power source configured to supply electrical energy to the aerosol-generating unit; and
a controller configured to control the supply of electrical energy from the power source to the aerosol-generating unit.

In some examples, the controller may be configured to control the supply of electrical energy from the power source to the aerosol-generating unit to supply a random pulse-width modulated voltage signal to the aerosol-generating unit. The random pulse-width modulated voltage signal may comprise toggling a signal between a high state and a low state (e.g., on and off). A random pulse-width modulated signal is different from conventional pulse-width modulated signals which involve toggling a signal between a high and a low state (e.g., on and off) according to a predictable/regular pattern. By providing a random pulse-width modulated signal, electromagnetic interference may be reduced.

The random pulse-width modulated voltage signal may comprise a plurality of voltage pulses, e.g. in a single cycle period (for example 20 ms). A voltage pulse may correspond to a high state (e.g., on). Between the voltage pulses, no electrical energy may be supplied, corresponding to a low state (e.g., off).

The random pulse-width modulated voltage signal may comprise a (regular) repeated cycle period, with a plurality of voltage pulses within each cycle period.

The random pulse-width modulated voltage signal may be characterized by one or more randomly generated parameters. The randomly generated parameters may be generated by the controller. For example, the one or more randomly generated parameters may include any one or any combination of: the number of voltage pulses in a predetermined period of time (e.g., cycle period), such as 20 ms, the position of one or more of the voltage pulses within the cycle period (or the position of each voltage pulse within the cycle period), the width of one or more of the voltage pulses (or the width of each voltage pulse), and/or the time between consecutive voltage pulses.

In some examples, the width of at least one (or each) pulse within the cycle period may be between 0.5 ms and 10 ms. That is, in some examples, the width of one or more of the pulses, or each pulse, may be randomly generated within predetermined limits such as from 0.5 ms to 15 ms. In some examples, the width of one or more of the pulses, or each pulse, may be randomly generated to be at least 0.5 ms, or at least 1 ms, or at least 5 ms, or at least 10 ms. Additionally, or alternatively, the width of one or more of the pulses, or each pulse, may be randomly generated to be 15 ms or less, or 10 ms or less, or 5 ms or less, or 1 ms or less.

The time between consecutive pulses may be between 0.5 ms and 10 ms. Thus, in some examples, the time between consecutive pulses may be randomly generated within predetermined limits such as from 0.5 ms to 10 ms.

In some examples, the random pulse-width modulated voltage signal may have a predetermined (e.g. constant) cycle period, for example of between 10 and 30ms, for example around 20 ms.

The random pulse-width modulated voltage signal may have between 2 and 10 voltage pulses per cycle period. In some examples, the number of voltage pulses per cycle period may be randomly generated within predetermined limits such as between 2 and 10.

In some examples, the random pulse-width modulated voltage signal may have a constant duty cycle over time. For example, the duty cycle may be around 20%, or around 30%, or around 40%, or around 50%, or around 60%, or around 70%, or around 80%.

The aerosol-generating system may comprise one or more sensors configured to perform measurements on the system to generate feedback data. For example, the one or more sensors may be configured to measure a metric corresponding to the temperature of the aerosol-generating unit. The one or more sensors may be configured to measure a resistance, current and/or voltage, e.g., of the aerosol-generating unit. The one or more sensors may be communicatively coupled to the controller. Thus, the controller may be configured to receive the feedback data (e.g., the measurements corresponding to temperature, resistance, current and/or voltage) from the one or more sensors.

In some examples, the controller may be configured to modify characteristics of the random pulse-width modulated voltage signal based on the received feedback data. For example, the controller may be configured to vary the duty cycle of the random pulse-width modulated voltage signal based on feedback (e.g. temperature of the aerosol-generating unit). Additionally, or alternatively, the controller may be configured to vary the predetermined lower and upper limits for the pulse width and/or the time between consecutive pulses based on the feedback data.

As used herein, a "controller" may refer to one or more units for processing data, examples of which may include an ASIC, microcontroller, FPGA, microprocessor, digital signal processor (DSP) capability, state machine or other suitable component. A controller may be configured to execute a computer program, e.g. which may take the form of machine-readable instructions, which may be stored on a non-transitory memory and/or programmable logic. The processing resource may have various arrangements corresponding to those discussed for the circuitry, e.g. on-board and/or off board the device as part of the system. As used herein, any machine executable instructions, or computer readable media, may be configured to cause a disclosed method (such as that of the second aspect) to be carried out, e.g. by an aerosol-generating system as disclosed herein, and may therefore be used synonymously with the term method.

The power source may include a battery. Suitable batteries may include lithium-based batteries (e.g. lithium-ion and lithium polymer batteries) and nickel-base batteries (e.g. nickel-cadmium and nickel-metal hydride batteries). The battery may be rechargeable via a wired or wireless connection. The power source may, alternatively or in addition, include an external power outlet configured to charge the battery, bypass the battery, or to replace the battery altogether. The power source may be or include another form of charge storage device such as a capacitor.

The aerosol-generating unit may be a heating system configured to generate heat upon being supplied with electrical power.

The heating system may be arranged as an outside-in heater arrangement in which a heating element is arranged in or on a side wall of the cavity of the aerosol-generating system into which the consumable in inserted. In this way, the heating element heats an outer surface of the consumable when inserted into the cavity.

The heating system may be arranged as an inside-out heater arrangement in which a heating element is positioned in the consumable. In this way, the heat from the heating element radiates out radially from within the consumable.

The heating element may be provided in several forms. The heating element may comprise a cylindrical or rod-shaped heating element configured for insertion into an end of the consumable. The heating element may comprise a blade-shaped heating element defining a generally planar heating element configured for insertion into an end of the consumable. The heating element may comprise a helically wound heating element (coil heating element), a mesh heating element comprising a plurality of interconnected cells that together define a heating area, e.g. formed by a perforated sheet or network of interleaved wires, or a heater track in the form of a conductive channel formed on a substrate. The heating element may comprise a susceptor formed of a material configured to absorb electromagnetic energy and convert it into heat. The heating element may comprise an annulus (e.g. tube) into which the consumable is insertable.

It will be appreciated that many of these terms overlap. For example, a rod heating element may comprise heater tracks or a coil that generate the heat radiated by the rod heating element, or a tube heater may be heated by a heater track formed thereon.

The heating element may have a length that is less than, or substantially the same as, an axial length of an aerosol-forming material, such that the heating element may only extend across and/or penetrate the aerosol-forming material, rather than other components of the consumable. The heating element may be between 8 mm and 40 mm long, preferably between 10 mm and 18mm, and more preferably between 12 mm and 16mm.

The heating system may generate heat via one or more of several modes. Heat may be generated by resistive heating in which a current is passed through the resistive material of the heating element (e.g. the material of a wire, coil, mesh or track). Heat may be generated by microwave heating in which microwaves are directed towards a material to be heated. Heat may be generated by inductive heating in which an electromagnetic field is generated by an inductor. The electromagnetic field generates electrical currents in a conductive material (susceptor) that induce eddy currents to heat up the conductive material. The susceptor may be positioned about the consumable (an outside-in heating arrangement) or positioned in the consumable (an inside-out heating arrangement). Heat may be generated by infrared heating in which infrared radiated in directed towards a material to be heated.

A resistive heating element may be formed from a variety of suitable materials that include, but not limited to metals, metal alloys, ceramics, ceramic metals, carbon-based materials, and composites thereof.

Examples of suitable metals and metal alloys include: silver, copper, nickel, titanium, tungsten, zirconium, tantalum, platinium group metals, stainless steel, nickel-, cobalt-, chromium-, aluminium-titanium- zirconium-, hafnium-, niobium-, molybdenum-, tantalum-, tungsten-, tin-, gallium-, manganese- and iron-containing alloys, and super-alloys based on nickel, iron, cobalt, stainless steel, and iron-manganese-aluminium based alloys.

The resistive heating element may comprise one or more electrically conductive tracks formed of the abovementioned metal and metal alloys.

Examples of suitable ceramics include silicon carbide, molybdenum disilicide, aluminium oxide, silicon nitride, titanium carbide. The ceramic may be doped or undoped.

In some embodiments, the heating element may be formed on or comprise an electrically insulating substrate. The electrically insulating substrate may be a ceramic (e.g. aluminum oxide or zirconia), polymer (e.g. polyimide), fibrous material (e.g. paper or cardboard), or other like material suitable for providing electrical insulation and mechanical support to the heating element.

The electrically insulating substrate may have a thermal conductivity of less than 40 W/mK, a thermal conductivity of less than 30 W/mK a thermal conductivity of less than 20 W/mK a thermal conductivity of less than 10 W/mK.

The heating system may comprise a susceptor configured to generate heat in the presence of an electromagnetic field. The susceptor may be formed in the shape of a rod, sheet, band, or like shape. Suitable materials include metals, ceramics, and carbon-based materials such as graphite. The metal may be a ferromagnetic metal, such as iron, stainless steel, nickel, or cobalt. The metal may be a non-ferromagnetic metal, such as aluminium, copper or gold. Suitable ceramic materials include zirconia, aluminium oxide, and silicon carbide. The susceptor may be formed of at least 50% ferro- or paramagnetic material, or at least 70% ferro- or para- magnetic material.

In some examples, the aerosol-generating system may be an electronic vapour product. Thus, the aerosol-generating unit may be configured to generate an aerosol from liquid or gel aerosol precursor.

In other examples, the aerosol-generating system may be a heat-not-burn device. Thus, aerosol-generating unit may be configured to generate an aerosol from a solid precursor such as tobacco.

In a second aspect, the present disclosure provides a method of performing power control on an aerosol-generating system. The aerosol-generating system may comprise an aerosol-generating unit and a power source electrically connected to the aerosol-generating unit.

The method may comprise:
generating a random pulse-width modulated voltage signal, wherein the random pulse-width modulated voltage signal is characterised by one or more randomly generated parameters; and
supplying the random pulse-width modulated signal from the power source to the aerosol-generating unit.

In this way, electrical energy is supplied from the power source to the aerosol-generating unit.

The method of the second aspect may be performed on an aerosol-generating system according to the first aspect. As such, the method of the second aspect may comprise any of the features described with reference to the first aspect above, unless such a combination is clearly impermissible or expressly avoided. For example, the random pulse-width modulated (PWM) signal may have any one or any combination of the features of the random pulse-width modulated signal described with reference to the first aspect. Conversely, the controller of the first aspect may be configured to perform any one or any combination of the steps described with reference to the method of the second aspect.

The random pulse-width modulated voltage signal may be supplied to the aerosol-generating unit during cycle periods (also referred to as PWM cycles) having a pre-determined duration, for example 20 ms. Within each cycle period, the voltage signal may have a constant duty cycle, such as 50%. Supplying the random pulse-width modulated signal to the aerosol-generating unit may include supplying a plurality of voltage pulses during each cycle period.

In some examples, the method may include an initial step of measuring the resistance of a heating element of the aerosol-generating unit. This may be done by measuring the voltage of a potential divider formed by the heating element and a sense resistor having a known resistance, e.g. 1.6 Ω. The sense resistor may be temporarily connected to the power source (e.g. battery), for example via a MOSFET, during the measurement. The resistance of the heating element may be calculated based on the measured voltage.

The method may further include determining instantaneous power, Pi, of the power source based on the measured resistance of the heating element, Rh, and the voltage of the power source (Vbatt). This may be calculated as: ${P}_{i} = \frac{{V}_{batt}^{2}}{{R}_{h}}$, in accordance with Ohm's law. When the resistance of the heating element equals 0.8 Ω, the instantaneous power Pi is between 12W and 20 W.

The method may further include a step of determining a target duty cycle of the voltage signal for supply to the heating element based on a target power value, the calculated instantaneous power, and the duration of the cycle period. For example, the target duty cycle can be calculated as the amount of time the voltage signal needs to be supplied to the aerosol-generating unit for in order to reach a pre-determined target average power (e.g. 6.6W), based on the known instantaneous power value Pi and the known cycle period duration (e.g. 20 ms).

The method may include subsequently generating the random pulse-width modulated voltage signal, the random pulse-width modulated voltage signal having the determined target duty cycle. In other words, the randomly generated parameter(s) (e.g. pulse duration and/or time between consecutive pulses) may be generated such that the plurality of voltage pulses during a cycle period add up to the predetermined duty cycle, e.g. 50%.

In some examples, the method may comprise performing measurements on the aerosol-generating system to generate feedback data. For example, performing measurements may include measuring one or more metrics corresponding to the temperature, resistance, voltage and/or current of the aerosol-generating unit. The method may include transmitting the feedback data to a controller of the system, configured to control the power source to supply the random pulse-width modulated voltage signal to the aerosol-generating unit.

The method may involve modifying characteristics of the random pulse-width modulated voltage signal, following its generation, based on the received feedback data. For example, the method may include varying the duty cycle of the random pulse-width modulated voltage signal based on feedback from an aerosol-generating unit of the system. Additionally, or alternatively, the method may include varying the predetermined lower and upper limits for the pulse width and/or the time between consecutive pulses based on the feedback data.

The preceding summary is provided for purposes of summarizing some examples to provide a basic understanding of aspects of the subject matter described herein. Accordingly, the above-described features should not be construed to narrow the scope of the subject matter described herein in any way. Moreover, the above and/or following examples may be combined in any suitable combination to provide further examples, except where such a combination is clearly impermissible or expressly avoided. Other features, aspects, and advantages of the subject matter described herein will become apparent from the following text and the accompanying figures.

### BRIEF DESCRIPTION OF THE FIGURES

Aspects, features and advantages of the present disclosure will become apparent from the following description of examples in reference to the appended figures in which like numerals denote like elements.
Figure 1 shows an example of an aerosol-generating system;
Figure 2 shows internal components of the aerosol-generating system;
Figure 3 shows a random pulse-width modulated voltage signal according to the present disclosure, and a conventional pulse-width modulated voltage signal;
Figure 4 shows a flow diagram of a method of performing power control on the aerosol-generating system of Figures 1 and 2.
Figure 5 shows an example of an aerosol-generating system;
Figure 6 shows the components of the aerosol-generating system in a 'disengaged state'; and
Figure 7 shows the components of the aerosol-generating system in an 'engaged state'.

### DETAILED DESCRIPTION OF EMBODIMENTS

It is to be understood that the present disclosure, which includes the specification and claim(s), is not limited by specific construction details or process steps. Rather, it will be clear to those skilled in the art that the systems, apparatuses, and methods described herein can be embodied and practiced in various alternative ways without departing from the scope of the invention.

Unless defined otherwise, scientific and technical terms used herein have their meanings commonly understood by those skilled in the art and that known techniques and procedures may be performed according to conventional methods.

In the present disclosure, the terms "a" and "an" may mean "one", "one or more", "at least one", and "one or more than one" unless the context clearly indicates otherwise. Likewise, plural terms shall include the singular unless otherwise required by context.

In the present disclosure, the term "or" means an inclusive "and/or" unless explicitly indicated to refer to alternatives only or unless the alternatives are mutually exclusive.

In the present disclosure, the terms "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless stated otherwise, the features of examples disclosed herein, and of the claims, may be integrated together in any suitable arrangement such that combinations of features are not limited by the described forms, particularly the form (e.g. numbering) of example(s), embodiment(s), or dependency of claim(s). This also applies to the phrase "in one example", "according to an example" and the like, which are merely a stylistic form of wording not to be construed as limiting the features to a separate embodiment. This is to say, a reference to 'an,' 'one,' or 'some' examples(s) may be a reference to any one or more, and/or all examples, or combination(s) thereof, disclosed. Also, similarly, reference to "the" example may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably.

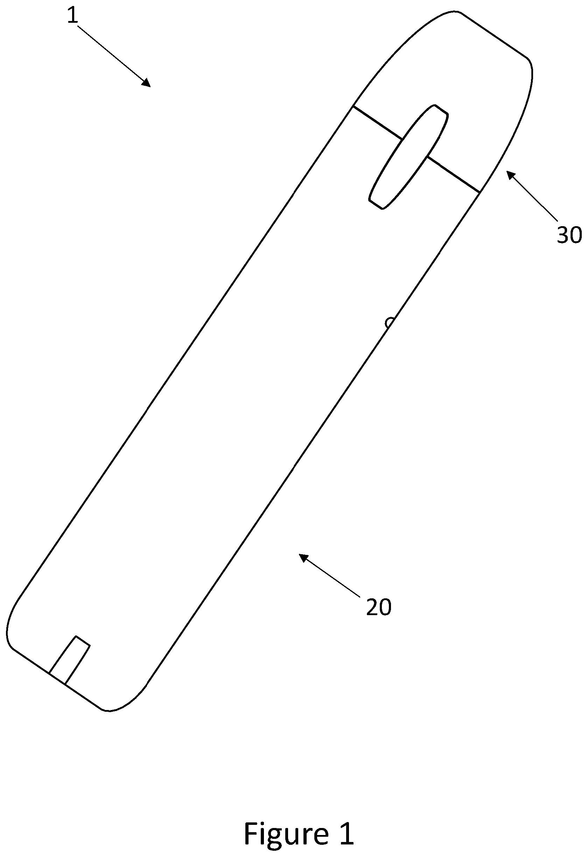

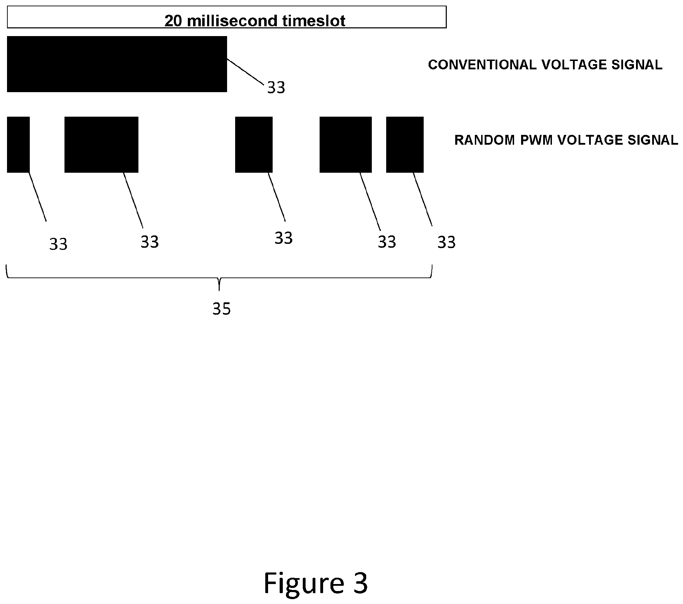

Figure 1 shows an example of an aerosol-generating system 1. In this example, the aerosol-generating system 1 is an electronic vapour product (EVP) configured to produce an aerosol from an aerosol-forming material 10 (e.g. a liquid or gel precursor), although it will be appreciated that the invention is similarly applicable to a heat-not-burn product (HNB) configured to produce an aerosol from heating an aerosol-forming material (e.g. a solid precursor such as tobacco) to a temperature below its combustion temperature, as discussed with reference to Figures 3-5 below.

The term "aerosol-forming material" refers to a substrate or formulation capable of releasing volatile components that can form an aerosol, e.g. by releasing volatile compounds in the aerosol-forming material. An "aerosol" is a dispersion of solid particles and/or liquid droplets dispersed in a gas. The aerosol may be visible or invisible.

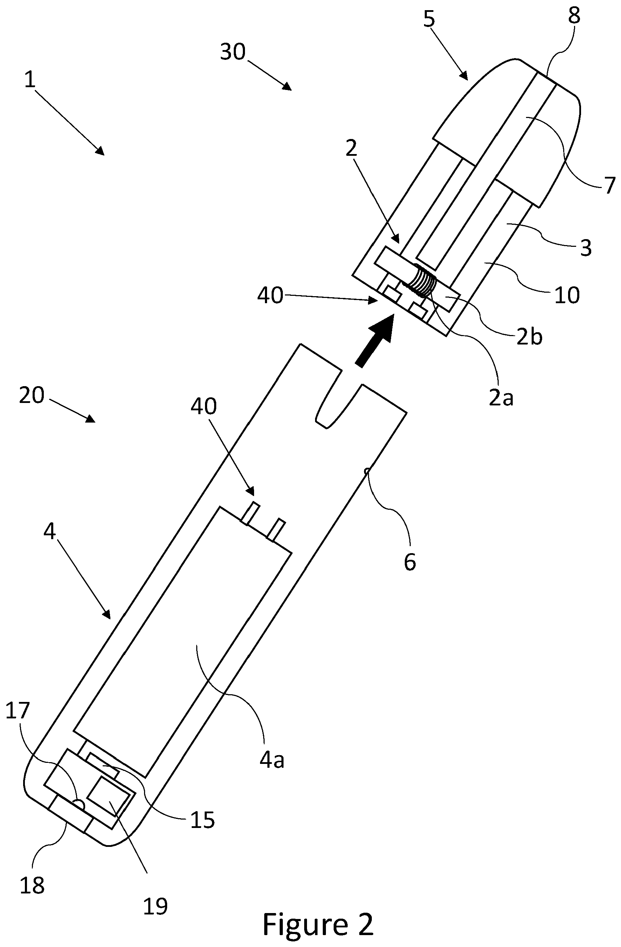

Figure 2 shows a schematic representation of the internal components of the aerosol-generating system 1. The aerosol-generating system 1 comprises an aerosol-generating unit 2 configured to generate an aerosol from an aerosol-forming material 10 held in a storage portion 3 of the aerosol-generating system 1 (implemented here as a "tank"). In this example, the aerosol-generating unit 2 is a heating system including a heating element 2a although it will be appreciated that an aerosol-generating unit comprising an ultrasonic element, atomiser or similar component may be provided.

The aerosol-forming material 10 may be referred to as "e-liquid". Typically, the aerosol-forming material 10 includes a base liquid and optionally nicotine and/or flavourings such that the resulting aerosol contains nicotine and/or flavourings.

The aerosol-generating system 1 includes a power source 4. In this example, the power source 4 includes a battery 4a configured to supply electrical energy to operate the aerosol-generating unit 2 and other components. The aerosol-generating system 1 may be powered, alternatively or in addition to the battery 4a, by an external power source.

The aerosol-generating system 1 includes an aerosol-delivery system 5 for delivery of the aerosol to a user. In this example, the aerosol-delivery system 5 comprises an air inlet 6, a mouthpiece 8, and an air passageway 7 extending therebetween via a region in proximity to the aerosol-generating unit 2.

The aerosol-generating unit 2 includes a wick 2b having at least one end that extends into the storage portion 3 and is configured to draw aerosol-forming material 10 out from the storage portion 3. In this example, the heating element 2a is in the form of a heating filament wrapped around a portion of the wick 2b. In this manner, the heating element 2a can heat up the aerosol-forming material 10 drawn out of the storage portion 3 by the wick 2b to produce the aerosol, which is then drawn through the air passageway 7 to the mouthpiece 8. In other examples, the heating element 2a may be a mesh heating element, ceramic heating element, or other means of generating heat. The heating element 2a may be part of an induction heating element having a susceptor configured to produce heat when penetrated by an alternating magnetic field.

The aerosol-generating system 1 may be a two-part construction comprising a device body 20 and an article 30 (alternatively referred to as a "pod" or "cartomizer") configured to be releasably connected/disconnected by an end user. The device body 20 and article 30 are each configured to house respective components of the aerosol-generating system 1.

In some examples, the article 30 may be configured to be disposed of upon depletion of aerosol-forming material 10 from the storage portion, in which case the article 30 may referred to as a "consumable". Alternatively, the article 30 may be configured for reuse, such that it is configured to be refillable upon depletion of the aerosol-forming material 10 from the storage portion.

In this example, the device body 20 comprises the power source 4, and the article 30 comprises the aerosol-generating unit 2 and storage portion 3, although it will be appreciated that the device body 20 and article 30 may house any respective set of components. The device body 20 and article 30 are configured to physically interlock to secure the article 30 relative to the device body 20.

Upon connection, electrical connectors 40 of the device body 20 and article 30 may establish an electrical connection between the device body 20 and the article 30. In this way, electrical power can be supplied from the power source 4 to the aerosol-generating unit 2 (or other components of the article 30) without the article 30 needing to have its own power source.

The device body 20 may include any one or more of electrical circuitry, a memory, a wireless interface, and one or more other components. The device body 20 may include a printed circuit board (PCB) 25 on which components of the electrical circuitry, memory, wireless interface, and other components may be mounted.

The wireless interface may be configured to communicate wirelessly with an external (e.g. mobile) device, e.g. via Bluetooth. The other component(s) may include one or more user interface devices configured to convey information to a user, a pressure sensor 15, and/or a charging port 18 (see e.g. figure 2).

The aerosol-generating system 1 may comprise one or more input and/or output elements. In this example, the aerosol-generating system 1 includes an input element in the form of a pressure sensor 15 arranged to detect a "puff" of the user, and particularly the resultant change in air pressure, i.e. a vacuum pressure generated by the user. Alternatively, or in addition, the aerosol-generating system 1 may include other means of detecting airflow, such as a flowmeter or microphone. The user can thereby activate the aerosol-generating unit 2 when inhaling through the mouthpiece 8. The aerosol-generating unit 2 creates an aerosol which is carried by the flow through the air passageway 7 and out of the mouthpiece 8.

The input and/or output elements may form part of a user interface (UI) of the aerosol-generating system 1. For instance, figure 2 shows an output element in the form of a light (e.g. an LED) 17. The light 17 is configured to convey information to the user regarding the state of the system 1. It will be appreciated that the input element(s) may be provided in various forms, such as touch screens, switches, and sensors, and the output element(s) may be provided in various forms, such as display screens, speakers, or a haptic output generated by a vibration generator.

The aerosol-generating system 1 further includes a controller 19 which is a processing resource for controlling one or more operations of the body 20 and article 30, e.g. based on instructions stored in the memory. The electrical circuitry may comprise the controller 19. As such, the controller 19 can be provided as part of the PCB 25.

In the present examples, the controller 19 is configured to control the power source 4 to supply a random pulse-width modulated voltage signal 35 to the aerosol-generating unit 2. The random pulse-width modulated voltage signal 35 is characterized by one or more randomly generated parameters. For example, the one or more randomly generated parameters may include any one or any combination of: the number of voltage pulses 33 in a predetermined period of time (e.g., cycle period), such as 20 ms, the position of each voltage pulse 33 within the cycle period, the width of each pulse 33, and/or the time between consecutive pulses 33.

An example of a random pulse-width modulated voltage signal 35 is shown in Figure 3, in comparison to a conventional voltage signal. In the example of Figure 3, the cycle period (timeslot) during which the voltage signal is supplied to the aerosol-generating unit 2 is 20 ms. The duty cycle of the voltage signal 35 is 50%. A conventional voltage signal having a duty cycle of 50% is shown on the top, with the black rectangle representing the time (i.e. 10 ms) during which the voltage signal is in an "on" state, i.e. being supplied to the aerosol-generating unit 2. Underneath the conventional voltage signal is shown an example of a random pulse-width modulated voltage signal 35 according to the present disclosure, also having a duty cycle of 50%. In this case, the voltage signal comprises a plurality of voltage pulses 33 (black rectangles) within the cycle period, each having a random pulse duration and being spaced by a random amount of time from its preceding and succeeding voltage pulses 33.

The width of each pulse 33 is between 0.5 ms and 15 ms. The time between consecutive pulses is between 0.5 ms and 10 ms.

A subsequent cycle period may have the same duty cycle (e.g., of 50%), but the number of pulses, position of each pulse, width of each pulse and/or time between consecutive pulses may be different by virtue of one or more of these parameters being randomly generated by the controller for each cycle period.

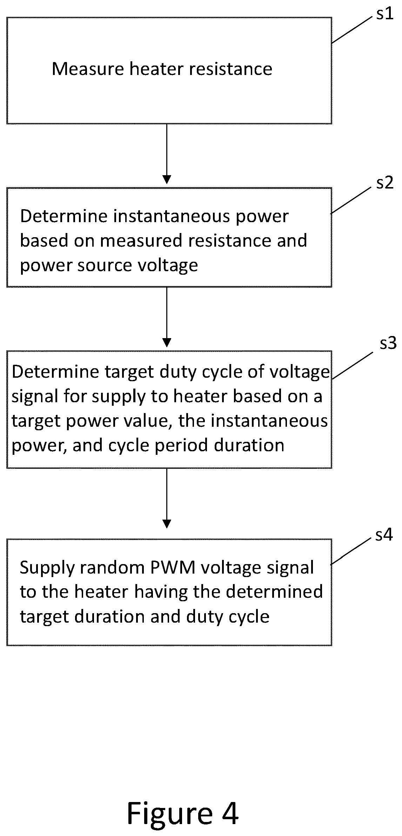

In some examples, the controller is configured to perform a power control method having the steps show in Figure 4.

In a first step s1, the controller 19 measures the resistance of the heating element 2a of the aerosol-generating unit 2. This is done by measuring the voltage of a potential divider formed by the heating element 2a and a sense resistor having a known resistance, e.g. 1.6 Ω. The sense resistor may be temporarily connected to the power source (e.g. battery), for example via a MOSFET, during the measurement. The resistance of the heating element 2a may be calculated based on the measured voltage.

Next, in step s2, the controller determines the instantaneous power, Pi, of the power source based on the measured resistance of the heating element 2a, Rh, and the voltage of the power source (Vbatt).

This may be calculated as: ${P}_{i} = \frac{{V}_{batt}^{2}}{{R}_{h}}$, in accordance with Ohm's law. When the resistance of the heating element 2a equals 0.8 Ω, the instantaneous power Pi is between 12W and 20 W.

In step s3, the controller determines a target duty cycle of the voltage signal 35 for supply to the heating element 2a based on a target power value, the calculated instantaneous power, and the duration of the cycle period. That is, the target duty cycle is calculated as the amount of time the voltage signal 35 needs to be supplied to the aerosol-generating unit 2 for in order to reach a pre-determined target average power (e.g. 6.6W), based on the known instantaneous power value Pi and the known cycle period duration (e.g. 20 ms).

The controller 19 subsequently generates the random pulse-width modulated voltage signal 35 in s4, the random pulse-width modulated voltage signal 35 having the determined target duty cycle. In other words, the randomly generated parameter(s) (e.g. pulse duration and/or time between consecutive pulses) are generated such that the plurality of voltage pulses during a cycle period add up to the predetermined duty cycle, e.g. 50%.

Steps s1-s4 may be continuously repeated.

Optionally, the method can also comprise performing one or more other measurements on the aerosol-generating system to generate feedback data. For example, the measurements may include measuring a metric related to the temperature of the aerosol-generating unit 2. The measurements may be performed by one or more sensors of the aerosol-generating system. The method can include transmitting the feedback data to the controller 19 of the system. The controller 19 may then modify characteristics of the random pulse-width modulated voltage signal 35, following its generation, based on the received feedback data. For example, the method may include varying the duty cycle of the random pulse-width modulated voltage signal 35 based on feedback from the aerosol-generating unit 2. Additionally, or alternatively, the method may include varying the predetermined lower and upper limits for the pulse width and/or the time between consecutive pulses 33 based on the feedback data.

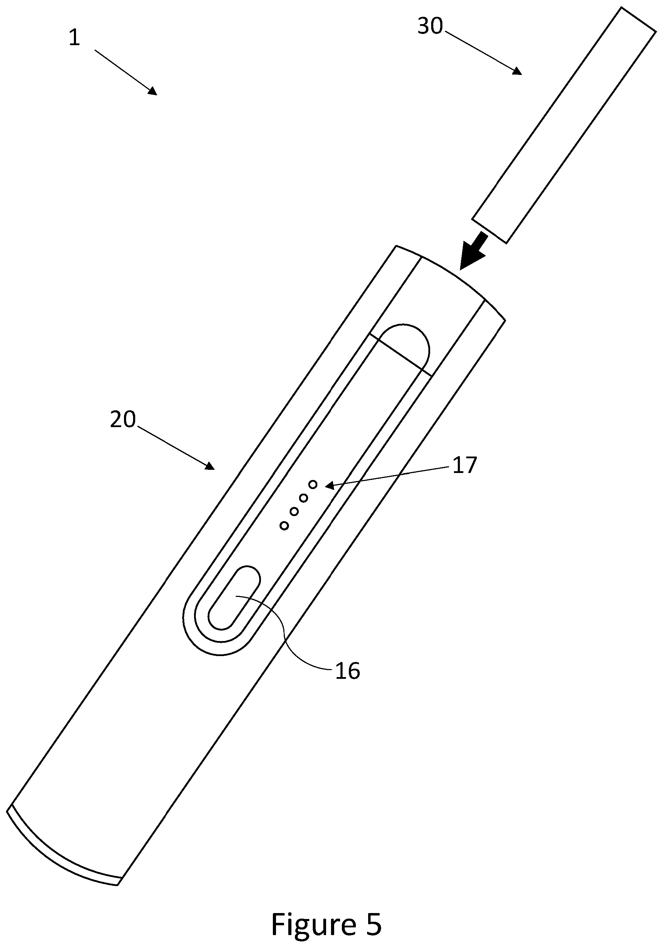

Instead of an EVP, the aerosol-generating system can be a heat-not-burn device instead. This is discussed with reference to Figures 5-7.

Figure 5 shows an example of an aerosol-generating system 1 comprising a device body 20 and an article 30 (alternatively referred to as a "consumable") for insertion into the device body 20. In this example, the device body 20 is a heat-not-burn device (HNB) configured to produce an aerosol by heating an aerosol-forming material 10 (e.g. a solid precursor, such as tobacco material) to a temperature below its combustion temperature.

The term "aerosol-forming material" refers to a material capable of releasing volatile components that can form an aerosol, e.g. by releasing volatile compounds in the aerosol-forming material 10. An "aerosol" is a dispersion of solid particles and/or liquid droplets dispersed in a gas. The aerosol may be visible or invisible.

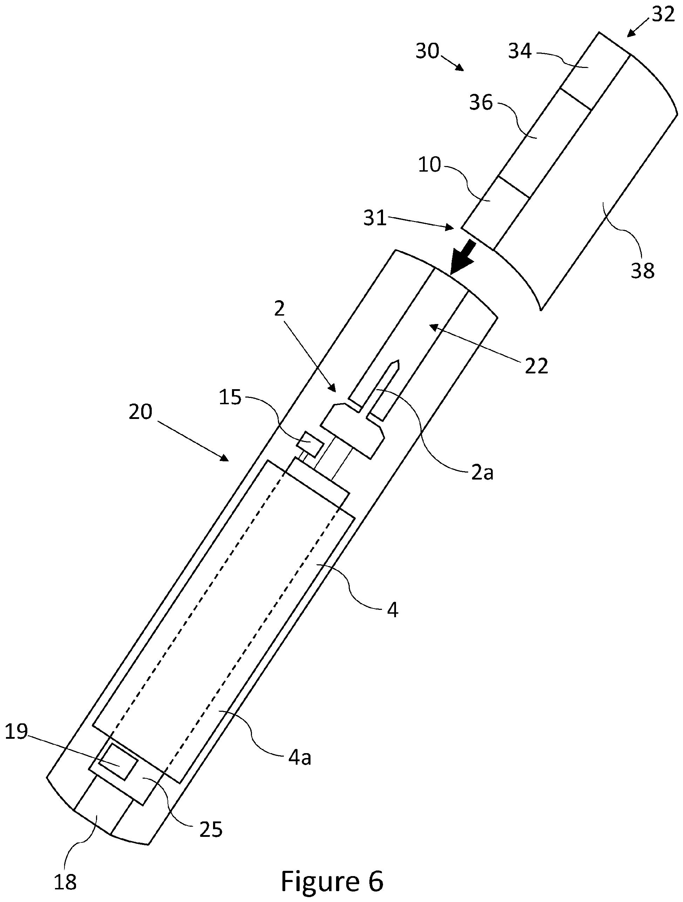

Figure 6 shows a schematic representation of the internal components of the device body 20 and article 30. The device body 20 and article 30 are shown in a "disengaged state" in which the article 30 is separated from the device body 20.

The aerosol-generating system 1 has an aerosol-generating unit 2 in the form of a heating system. The heating system comprises a heating element 2a configured to penetrate the article 30. The heating element 2a heats the aerosol-forming material 10 to a temperature lower than its combustion temperature such that an aerosol can be formed by evaporation, torrefaction and pyrolysis.

As in the EVP of Figures 1 and 2, the aerosol-generating system 1 of Figures 5-7 includes a power source 4 which includes a battery 4a configured to supply electrical energy to operate the aerosol-generating unit 2 and other components. The aerosol-generating system 1 may be powered, alternatively or in addition to the battery 4a, by an external power source.

The article 30 defines an air passageway which transmits aerosol generated from the aerosol-forming material 10 to a downstream end 32 (i.e. terminal end or mouth end) of the article 30. The article 30 may comprise one or more filter elements 34, one or more spacer elements 36, and one or more wrapping layers 38 (e.g. paper, foil or composite layers). One or more of the filter elements 34 and spacer elements 36 may define the air passageway. In the present example, the article 30 comprises an aerosol-forming material 10 at the upstream end 31 of the article 30, a filter element 34 at the downstream end 32, and a spacer element 36 positioned between them, all circumscribed by a wrapping layer 38.

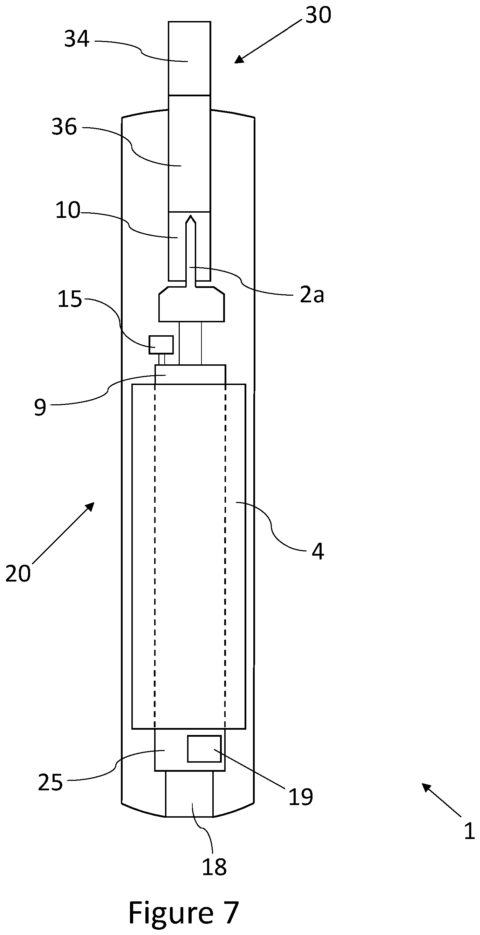

Figure 7 shows the aerosol-generating system 1 in an "engaged state" in which the article 30 is inserted into an internal chamber 22 of the device body 20.

In this example, the heating element 2a is configured to penetrate the article 30. The heating element 2a is arranged to extend along a length of the aerosol-forming material 10 when inserted therein, such that when the heating element 2a is activated, heat is transferred radially outward from the heating element 2a to the aerosol-forming material 10 resulting in formation of an aerosol. This may be referred to as an 'inside-out' heating arrangement. The aerosol is subsequently entrained in an airflow produced by the action of the user drawing on the downstream end 32 of the article 30.

In alternative examples, the heating element 2a may be configured to at least partially encircle the article 30, such that when the heating element 2a is activated, heat is transferred radially inward from the heating element 2a to the aerosol-forming material 10. This may be referred to as an 'outside-in' heating arrangement.

In this example, the heating element 2a is a resistive heating element although it will be appreciated that the aerosol-forming material 10 may be heated by any suitable means. For instance, the heating element 2a may comprise a susceptor (not shown) configured to produce heat when penetrated by an alternating magnetic field.

The device body 20 may include any one or more of electrical circuitry, a memory, a wireless interface, and one or more other components. The device body 20 may include a printed circuit board (PCB) 25 on which components of the electrical circuitry, memory, wireless interface, and other components may be mounted.

The aerosol-generating system 1 may comprise one or more input and/or output elements. The input and/or output elements may form part of a user interface (UI) of the system 1. For instance, figure 1 shows an input element in the form of a button 16 and an output element in the form of a set of lights (LEDs) 17. The button 16 is configured to control at least one function of system 1, such as power source to the heating element 2a. The lights 17 are configured to convey information to the user regarding the state of the system 1 and/or article. It will be appreciated that the input element(s) may be provided in various forms such as touch screens, switches, and sensors, and the output element(s) may be provided in various forms such as display screens, speakers, or a haptic output generated by a vibration generator.

The HNB device of Figures 5-7 also includes a controller 19 having the same functionality as that described in relation to Figures 1-4 above.

## Claims

1. An aerosol-generating system that comprises:
an aerosol-generating unit configured to generate an aerosol from an aerosol-forming material;
a power source configured to supply electrical energy to the aerosol-generating unit; and
a controller configured to control the supply of electrical energy from the power source to the aerosol-generating unit to supply a random pulse-width modulated voltage signal to the aerosol-generating unit, the random pulse-width modulated voltage signal comprising a plurality of voltage pulses and being **characterized by** one or more randomly generated parameters.

2. The aerosol-generating system of claim 1, wherein:
the one or more randomly generated parameters includes any one or any combination of: the number of voltage pulses in a predetermined period of time, the position of one or more of the voltage pulses, the width of one or more of the voltage pulses, and/or the time between consecutive voltage pulses.

3. The aerosol-generating system of claim 1 or 2 wherein the width of at least one of the voltage pulses is between 0.5 ms and 15 ms.

4. The aerosol-generating system of any one of the preceding claims, wherein a time between consecutive voltage pulses is between 0.5 ms and 10 ms.

5. The aerosol-generating system of any one of the preceding claims, wherein the random pulse-width modulated voltage signal has a constant cycle period.

6. The aerosol-generating system of claim 5, wherein the constant cycle period is approximately 20 ms.

7. The aerosol-generating system of any one of the preceding claims, wherein the random pulse-width modulated voltage signal has between 2 and 10 voltage pulses per cycle period.

8. The aerosol-generating system of any one of the preceding claims, wherein the random pulse-width modulated voltage signal has a constant duty cycle over time.

9. The aerosol-generating system of any one of the preceding claims, wherein the aerosol-generating system comprises one or more sensors configured to perform measurements on the aerosol-generating system to generate feedback data.

10. The aerosol-generating system of claim 9, wherein the controller is communicatively coupled to the one or more sensors to receive the feedback data therefrom, and the controller is configured to modify one or more characteristics of the random pulse-width modulated voltage signal based on the received feedback data.

11. The aerosol-generating system of claim 10, wherein the controller is configured to vary the duty cycle of the random pulse-width modulated voltage signal and/or predetermined lower and upper limits for the pulse width and/or the time between consecutive pulses based on the feedback data.

12. The aerosol-generating system of any one of the preceding claims, wherein the aerosol-generating system is an electronic vapour product.

13. The aerosol-generating system of any one of claims 1 to 11, wherein the aerosol-generating system is a heat-not-burn device.

14. A method of performing power control on an aerosol-generating system, the aerosol-generating system comprising an aerosol-generating unit and a power source electrically connected to the aerosol-generating unit, the method comprising:
generating a random pulse-width modulated voltage signal, wherein the random pulse-width modulated voltage signal is **characterised by** one or more randomly generated parameters; and
supplying the random pulse-width modulated signal from the power source to the aerosol-generating unit.
